(19) Europäisches Patentamt / European Patent Office / Office européen des brevets

(11) **EP 3 525 161 A1**

(12) **EUROPEAN PATENT APPLICATION**
published in accordance with Art. 153(4) EPC

(43) Date of publication:
**14.08.2019 Bulletin 2019/33**

(21) Application number: **17858045.2**

(22) Date of filing: **20.07.2017**

(51) Int Cl.:
*G06Q 50/22* [(2018.01)]    *A61B 5/16* [(2006.01)]
*A61B 5/00* [(2006.01)]    *A61B 5/11* [(2006.01)]
*A61B 5/1171* [(2016.01)]

(86) International application number:
**PCT/JP2017/026329**

(87) International publication number:
**WO 2018/066205 (12.04.2018 Gazette 2018/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **06.10.2016 JP 2016198527**

(71) Applicants:
 • **Sony Corporation**
  **Tokyo 108-0075 (JP)**
 • **Sony Mobile Communications Inc.**
  **Shinagawa-ku**
  **Tokyo 140-0002 (JP)**

(72) Inventors:
 • **SAKAI, Shimon**
  **Tokyo 108-0075 (JP)**
 • **HOSOKAWA, Satoshi**
  **Tokyo 140-0002 (JP)**
 • **NAKAGAWA, Ayumi**
  **Tokyo 108-0075 (JP)**

(74) Representative: **MFG Patentanwälte Meyer-Wildhagen Meggle-Freund Gerhard PartG mbB Amalienstraße 62 80799 München (DE)**

(54) **INFORMATION PROCESSING DEVICE AND INFORMATION PROCESSING METHOD**

(57)    [Object] To provide an information processing apparatus and an information processing method.
    [Solution] The information processing apparatus includes: an acquisition unit configured to acquire a fatigue level of a user; and a proposal generation unit configured to generate proposal information for fatigue improvement on the basis of the fatigue level.

**FIG. 1**

**Description**

Technical Field

The present disclosure relates to an information processing apparatus and an information processing method.

Background Art

Estimation of fatigue is performed. For example, Patent Literature 1 discloses a technology of measuring and evaluating eye fatigue of a subject by measuring a time frequency characteristic of a visual system in display observation, and measuring a variation amount of a threshold value level of the time frequency response.

Citation List

Patent Literature

Patent Literature 1: JP H10-024017A

Disclosure of Invention

Technical Problem

In the above-described technology, for example, fatigue of a user can be estimated, but it has been difficult to improve the fatigue of the user. In view of the foregoing, the present disclosure proposes an information processing apparatus and an information processing method that are novel and improved, and can perform proposal for improving fatigue.

Solution to Problem

According to the present disclosure, there is provided an information processing apparatus including: an acquisition unit configured to acquire a fatigue level of a user; and a proposal generation unit configured to generate proposal information for fatigue improvement on the basis of the fatigue level.
In addition, according to the present disclosure, there is provided an information processing apparatus including: a communication unit configured to transmit user information regarding a user, and receive proposal information for fatigue improvement; and a processing unit configured to perform processing that is based on the proposal information.
In addition, according to the present disclosure, there is provided an information processing method including: acquiring a fatigue level; and generating, by a processor, proposal information for fatigue improvement on the basis of the fatigue level.
In addition, according to the present disclosure, there is provided an information processing method including: transmitting user information regarding a user, and receiving proposal information for fatigue improvement; and performing, by a processor, processing that is based on the proposal information.

Advantageous Effects of Invention

As described above, according to the present disclosure, proposal for improving fatigue can be performed.
Note that the effects described above are not necessarily limitative. With or in the place of the above effects, there may be achieved any one of the effects described in this specification or other effects that may be grasped from this specification.

Brief Description of Drawings

FIG. 1 is a block diagram illustrating a configuration example of an information processing apparatus according to an embodiment of the present disclosure.
FIG. 2 is an example of a feedback request screen displayed on a display included in an output unit 40 according to the embodiment.
FIG. 3 is a conceptual diagram illustrating an example of proposal information generated by a proposal generation unit 14 according to the embodiment on the basis of a fatigue level of each fatigue type.

FIG. 4 is a flowchart diagram illustrating an example of a flow of processing of an information processing apparatus 1 according to the embodiment.

FIG. 5 is a conceptual diagram illustrating a specific example of improvement proposal for a user U1 according to the embodiment.

FIG. 6 is a conceptual diagram illustrating a specific example of improvement proposal for a user U2 according to the embodiment.

FIG. 7 is an explanatory diagram illustrating a configuration of an information processing system 99 according to a first modified example of the embodiment.

FIG. 8 is a block diagram illustrating a configuration example of a client terminal 2 according to the modified example.

FIG. 9 is a block diagram illustrating a configuration example of a server 3 according to the modified example.

FIG. 10 is a sequence diagram illustrating an operation example of the information processing system 99 according to the modified example.

FIG. 11 is an explanatory diagram illustrating a hardware configuration example.

Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that the description will be given in the following order.

<<1. Configuration example>>

    <1-1. Overall configuration>
    <1-2. Details of control unit>

<<2. Operation example>>

    <2-1. Flow of processing>
    <2-2. Specific example of improvement proposal>

<<3. Modified example>>

    <3-1. First modified example>
    <3-2. Second modified example>

<<4. Hardware configuration example>>
<<5. Conclusion>>

<<1. Configuration example>>

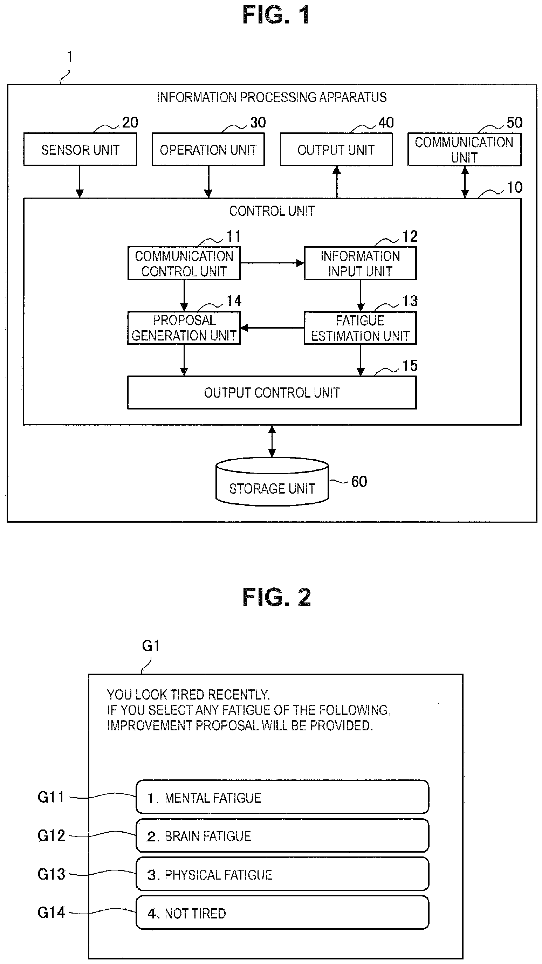

First of all, a configuration example of an embodiment of the present disclosure will be described with reference to FIG. 1. FIG. 1 is a block diagram illustrating a configuration example of an information processing apparatus according to an embodiment of the present disclosure.

As illustrated in FIG. 1, an information processing apparatus 1 according to the present embodiment includes a control unit 10, a sensor unit 20, an operation unit 30, an output unit 40, a communication unit 50, and a storage unit 60. The information processing apparatus 1 according to the present embodiment may be a smartphone, a personal computer (PC), a robot, a wearable device, or the like, for example. Hereinafter, an overall configuration of the information processing apparatus 1 will be described, and then, the control unit 10 will be described in detail.

<1-1. Overall configuration>

The control unit 10 controls each configuration of the information processing apparatus 1. For example, the control unit 10 estimates a fatigue level of a user on the basis of user information such as sensor information regarding the user that is input from the sensor unit 20, and information regarding an application used by the user that is received by the communication unit 50. In addition, the control unit 10 generates proposal information for fatigue improvement on the basis of the estimated fatigue level of the user, and causes the output unit 40 to output proposal for fatigue improvement. Note that a detailed configuration of the control unit 10 will be described later.

The sensor unit 20 provides the control unit 10 with information regarding the user (user information) that has been obtained by sensing a surrounding situation of the information processing apparatus 1. For example, the sensor unit 20 according to the present embodiment may include a microphone that generates a voice signal by sensing a voice of the user, a camera (or a depth camera) that acquires an image by image capturing, a human sensor that detects a human, a GPS sensor that acquires position information of the apparatus by receiving a GPS signal, and the like. Note that the sensor unit 20 is not limited to the above-described sensors, and may include various types of sensors such as an acceleration sensor, a gyro sensor, a geomagnetic sensor, a light sensor, a sound sensor, and a force sensor.

The operation unit 30 receives an operation input of the user, and provides the control unit 10 with the operation input. For example, the operation unit 30 according to the present embodiment may be implemented by a mouse, a keyboard, a touch panel, a button, a switch, a visual line input device, a gesture input device, a voice input device, or the like.

The output unit 40 performs an output in accordance with the control of the control unit 10. For example, the output unit 40 according to the present embodiment may include a display that can display and output characters, images, and other types of visual information, and a voice output device that can output a voice, such as a speaker and headphones. Note that the output unit 40 is not limited to the above devices and may be implemented by including a lamp that outputs light, a vibration device that outputs vibration, or the like.

The communication unit 50 performs information communication with another apparatus. By communicating information with another apparatus on the basis of the control of the control unit 10, the communication unit 50 can receive information regarding the user (user information) from the other apparatus, for example. Examples of user information received by the communication unit 50 will be described later.

The storage unit 60 stores programs and parameters for each configuration of the information processing apparatus 1 functioning. In addition, the storage unit 60 may store information regarding an application executed by the information processing apparatus 1, user information obtained in the past, a user profile (to be described later) generated on the basis of user information, a history of proposal information, and the like.

<1-2. Details of control unit>

The overall configuration example of the information processing apparatus 1 according to the present embodiment has been described above. Subsequently, a functional configuration of the control unit 10 included in the information processing apparatus 1 will be described in more detail.

As illustrated in FIG. 1, the control unit 10 according to the present embodiment has functions as a communication control unit 11, an information input unit 12, a fatigue estimation unit 13, a proposal generation unit 14, and an output control unit 15.

The communication control unit 11 controls communication performed by the communication unit 50. For example, the communication control unit 11 controls the communication unit 50 to receive user information.

The information input unit 12 acquires user information from the communication control unit 11, the sensor unit 20, and the storage unit 60, and inputs the acquired user information to the fatigue estimation unit 13 in a case where there is a change in the user information. For example, the information input unit 12 may compare the acquired user information and user information acquired in the previous time, and determine that there is a change in the user information, in a case where there is a difference satisfying a predetermined condition. The predetermined condition may be such a condition that the difference is larger than a threshold value, for example.

The user information acquired by the information input unit 12 may include, for example, position information of the user, information regarding an application used by the user, an action history of the user, state information of the user, and the like.

In a case where the information processing apparatus 1 is a device (e.g. smartphone, etc.) carried by the user, for example, the position information of the user may be acquired from the sensor unit 20. In addition, the position information of the user may be acquired from another device carried by the user, via the communication unit 50.

In addition, the information regarding an application used by the user may include schedule information (past schedule information and future schedule information) related to a schedule application, for example. In addition, the information regarding an application used by the user may be acquired from another apparatus via the communication unit 50, or may be acquired from the storage unit 60.

For example, in a case where the information regarding an application includes schedule information, the information input unit 12 may extract and acquire a schedule related to an increase or a decrease of a fatigue level, by keyword matching or the like. The schedule related to an increase or a decrease of a fatigue level may be a business schedule (normal work, business trip, etc.), a drinking party, travel, or the like, for example.

The action history of the user may include information such as bedtime, wake-up time, outgoing time, and return home time, for example. The action history of the user may be acquired from another apparatus via the communication unit 50, or may be estimated by the information input unit 12 on the basis of information acquired from the sensor unit 20 (acceleration information, position information of the user, etc.).

The state information of the user may include a face image, a smile frequency change, a voice pitch/intonation change, an attitude change, a sigh frequency, and the like, for example. The state information of the user may be acquired from the sensor unit 20, for example, or may be acquired by the information input unit 12 on the basis of information acquired from the sensor unit 20, and past user information stored in the storage unit 60.

For example, a face image can be acquired by performing face detection processing on an image acquired by a camera included in the sensor unit 20. In addition, the smile frequency change can be acquired on the basis of a result obtained by performing smile recognition processing on the detected face image, and a past smile recognition result. In addition, the voice pitch/intonation change can be acquired on the basis of voice data acquired by a microphone included in the sensor unit 20, and past voice data. In addition, the attitude change can be acquired on the basis of attitude data estimated from depth data acquired by a depth camera included in the sensor unit 20, and past attitude data. In addition, the sigh frequency can be acquired on the basis of sigh data detected on the basis of the face image and the voice data, and past sigh data.

In a case where user information is input from the information input unit 12, the fatigue estimation unit 13 (an example of an acquisition unit) estimates and acquires a fatigue level of the user on the basis of the user information. The fatigue estimation unit 13 may estimate and acquire a fatigue level of each fatigue type, for example. Examples of the fatigue type may include mental fatigue, brain fatigue, and physical fatigue.

The fatigue estimation unit 13 may estimate a fatigue level of each fatigue type by a fatigue estimator, which will be described below, for example.

When a past schedule is denoted by [(ps1, t1), (ps2, t2), ..., (psn, tn)], a future schedule is denoted by [fs1(t1), fs2(t2), ..., fso(to)], bedtime is denoted by [st1, st2, ..., stp], wake-up time is denoted by [wt1,wt2, ..., wtq], outgoing time is denoted by [ot1, ot2, ..., otr], return home time is denoted by [it1, it2, ...,its], a face image is denoted by face_image, a smile frequency change is denoted by lf(t), a voice pitch/intonation change is denoted by v(t), and an attitude change is denoted by p(t), a vector $(F_p, F_b, F_c)$ of a fatigue level of each fatigue type is estimated by a fatigue estimation function f (fatigue estimator) having the following parameters as inputs.

[Math. 1]

$$(F_p, F_b, F_c)=f([[(ps1, t1),(ps2, t2), \cdots, (psn, tn)], [fs1(t1), fs2(t2), \cdots, fso(to)],$$
$$[st1, st2, \cdots, stp], [wt1, wt2, \cdots, wtq], [ot1, ot2, \cdots, otr], [it1, it2, \cdots, its],$$
$$face\_image, lf(t), v(t), p(t))) \cdots (1)$$

In Formula (1) described above, $F_p$ denotes a fatigue level of mental fatigue, $F_b$ denotes a fatigue level of brain fatigue, and $F_c$ denotes a fatigue level of physical fatigue.

The fatigue estimator may be obtained by machine learning that uses learning data, for example. Here, a method of machine learning is not particularly limited.

The fatigue estimation unit 13 estimates and acquires a fatigue level of each fatigue type using the above-described fatigue estimator. In a case where an estimated fatigue level exceeds a threshold value, the fatigue estimation unit 13 provides a fatigue level of each fatigue type to the proposal generation unit 14 or the output control unit 15. In a case where any one of fatigue levels of the respective fatigue types exceeds a threshold value, for example, the fatigue estimation unit 13 may provide the fatigue levels of the respective fatigue types, or in a case where a total value of the fatigue levels of the respective fatigue types exceeds a threshold value, the fatigue estimation unit 13 may provide the fatigue levels of the respective fatigue types.

In addition, the fatigue estimation unit 13 may personalize the fatigue estimator on the basis of a user feedback regarding fatigue. For example, the fatigue estimation unit 13 personalizes the fatigue estimator for each user by further performing learning on the basis of a user feedback regarding fatigue that is obtained for each user. With this configuration, fatigue estimation accuracy of each user is enhanced.

For example, a user feedback regarding fatigue may be obtained by a feedback request to the user that is performed by the output unit 40, and an input operation of the user that is performed via the operation unit 30. For example, in a case where a fatigue level of each fatigue type is provided from the fatigue estimation unit 13, the output control unit 15 to be described later may control the output unit 40 to output a feedback request to the user.

FIG. 2 is an example of a feedback request screen displayed on a display included in the output unit 40. By the user pressing any of buttons G11 to G14 by operating the operation unit 30 on a screen G1 illustrated in FIG. 2, a user feedback is obtained. In the example illustrated in FIG. 2, information regarding a fatigue type selected by the user as a most applicable fatigue type is obtained as a user feedback.

In addition, the fatigue estimation unit 13 may personalize the fatigue estimator for each user by learning a corrected fatigue level of each type that has been obtained by correcting a fatigue estimation result, on the basis of the obtained user feedback.

For example, in the fatigue estimation result, when a fatigue level estimated value of a type having the largest fatigue level estimated value is denoted by $F_m$, and a fatigue level estimated value of a type selected in a user feedback is denoted by $F_u$, a corrected fatigue level $CF_u$ of the type selected by the user is represented by the following formula using a coefficient $\gamma = F_m / F_u$.

$$CF_u = F_u \times \alpha \times \gamma \ ... \ (2)$$

In Formula (2), a coefficient $\alpha$ (>0) is such a coefficient that a coefficient approaches 1 in accordance with time or the number of personalizations.

In addition, a corrected fatigue level $CF_n$ of a type unselected by the user is represented by the following formula using a fatigue level estimated value $F_n$ of a type unselected by the user.

$$CF_n = F_n \times \beta \ ... \ (3)$$

In Formula (3), a coefficient $\beta$ (>0) is such a coefficient that a coefficient approaches 1 in accordance with time or the number of personalizations.

By performing the correction of the fatigue estimation result in the above-described manner, a fatigue level of the type selected by the user becomes the largest value, and influence of correction becomes weaker as learning proceeds. Note that a correction method of a fatigue estimation result is not limited to the above method. Another correction method of a fatigue estimation result will be described later as a modified example.

In addition, a corrected fatigue level that is based on a user feedback may be not only used for learning for personalization but also provided to the proposal generation unit 14 as a fatigue level used for proposal information generation.

In addition, the fatigue estimation unit 13 may determine whether the fatigue estimator has been sufficiently personalized or not, by comparing the obtained user feedback and a fatigue estimation result obtained using the fatigue estimator. For example, the fatigue estimation unit 13 may determine that the fatigue estimator has been sufficiently personalized, in a case where a consistency probability in a predetermined number of comparison results obtained most recently exceeds a threshold value.

In a case where it is determined that the fatigue estimator has not been sufficiently personalized, the fatigue estimation unit 13 may provide a fatigue level of each fatigue type to the output control unit 15. Then, the output control unit 15 controls the output unit 40 to display the feedback request screen illustrated in FIG. 2, for example, whereby a user feedback regarding fatigue can be obtained.

In a case where it is determined that the fatigue estimator has been sufficiently personalized, the fatigue estimation unit 13 may provide a fatigue level of each fatigue type to the proposal generation unit 14 without providing a fatigue level of each fatigue type to the output control unit 15. With this configuration, in a case where the fatigue estimator has been sufficiently personalized, a feedback request to the user is skipped and a burden on the user is reduced.

The proposal generation unit 14 generates proposal information related to proposal for improving (reducing) fatigue, on the basis of the fatigue level of each fatigue type that has been provided by the fatigue estimation unit 13. The proposal information may include voice data, text data, image data, and the like, for example, and is provided from the proposal generation unit 14 to the output control unit 15 and output from the output unit 40 by a voice, display, or the like, in accordance with the control of the output control unit 15.

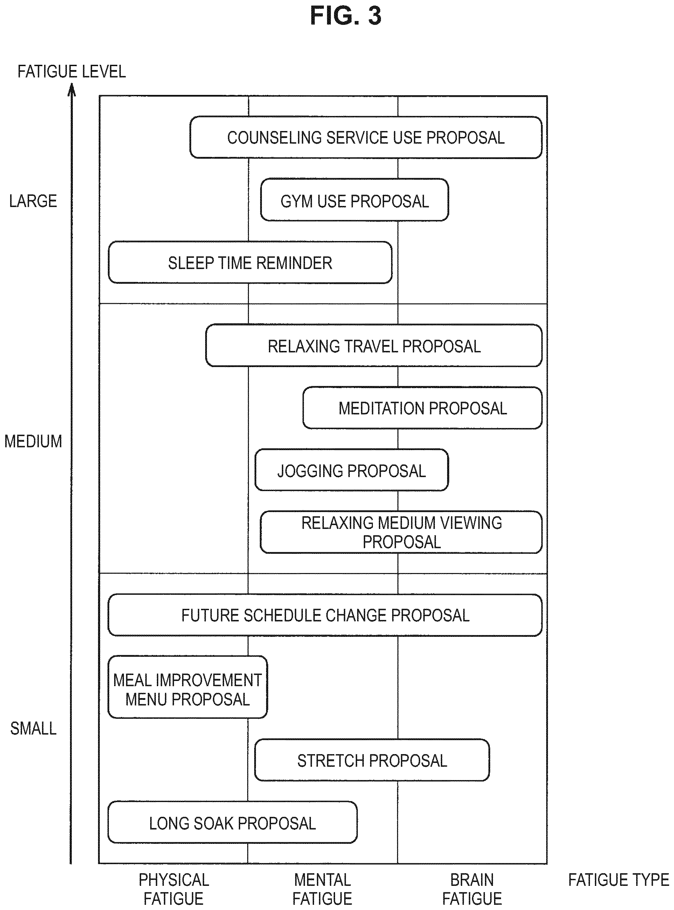

For example, the proposal generation unit 14 may generate proposal information on the basis of a fatigue type related to the largest fatigue level, and a value of the largest fatigue level. FIG. 3 is a conceptual diagram illustrating an example of proposal information generated by the proposal generation unit 14 on the basis of a fatigue level of each fatigue type.

A horizontal axis in FIG. 3 indicates a fatigue type related to the largest fatigue level, and a vertical axis in FIG. 3 indicates a value of the largest fatigue level. Note that the values of a large fatigue level may be classified into large, medium, and small as illustrated in FIG. 3, by threshold value processing, for example.

Meal improvement menu proposal illustrated in FIG. 3 may be proposal of meal menu containing deficient nutrient corresponding to a fatigue type (e.g. physical strength restoration menu containing amino acid), for example. In addition, future schedule change proposal may be proposal for extracting a schedule (e.g. "drinking party") that can increase a fatigue level, from a future schedule, and changing the schedule, for example. In addition, relaxing medium viewing proposal may be proposal for reproduction of relaxing video content or music content.

In addition, counseling service use proposal may be use proposal of counseling conducted by a doctor or the like, for example. Note that, in a case where user information in the counseling and a counseling result can be acquired, the fatigue estimation unit 13 may use, for personalization, the user information in the counseling and the counseling result, as a user feedback regarding fatigue.

In addition, the proposal generation unit 14 may generate proposal information further on the basis of a user profile related to the user. For example, the user profile may include information such as hobby, taste, a character, and a property of the user. The user profile may be estimated and obtained from application information, conversation (speech) of the user, or the like, or may be obtained by being input by the user. With this configuration, it becomes easier to perform fatigue improvement proposal more preferable for the user.

In addition, the proposal generation unit 14 may generate proposal information further on the basis of a history of the generated proposal information. For example, the proposal generation unit 14 may generate proposal information so as not to include proposal information included in the history (proposal information generated for the user in the past). In addition, the proposal generation unit 14 may generate proposal information so as not to include proposal information related to proposal output to the user in a predetermined most recent period or a predetermined number of most recent proposals. With this configuration, it becomes possible to suppress mannerism of fatigue improvement proposal.

Note that the history of proposal information may include information as to whether proposal that is based on the proposal information has been actually implemented by the user or not. The information as to whether proposal that is based on the proposal information has been actually accepted (implemented) by the user or not may be obtained by acquiring a user feedback for the proposal, for example. For example, a feedback for the proposal may be acquired by the user performing an input via the operation unit 30 after the output control unit 15 outputs proposal on the basis of proposal information.

The output control unit 15 controls an output of the output unit 40. For example, in a case where a fatigue level is provided from the fatigue estimation unit 13, the output control unit 15 may cause the fatigue level of each fatigue type (fatigue estimation result) provided by the fatigue estimation unit 13, to be output by a voice output, a display output, or the like. In addition, in a case where a fatigue level is provided from the fatigue estimation unit 13, the output control unit 15 may cause a feedback request regarding fatigue, to be output.

In addition, the output control unit 15 may cause fatigue improvement proposal to be output on the basis of proposal information provided from the proposal generation unit 14. For example, in a case where proposal information includes voice data, the output control unit 15 may cause the proposal information to be output by voice on the basis of the voice data. In addition, in a case where proposal information includes text data, the output control unit 15 may cause the text data to be output by display, or may convert the text data into voice data and then cause the voice data to be output by voice. In addition, in a case where proposal information includes image data, the output control unit 15 may cause the image data to be output by display.

The configuration of the information processing apparatus 1 according to the present embodiment has been specifically described above. Note that the configuration of the information processing apparatus 1 illustrated in FIG. 1 is an example, and the present embodiment is not limited to this. For example, each function of the control unit 10 according to the present embodiment may be included in another information processing apparatus connected via the communication unit 50. This example will be described later as a modified example.

<<2. Operation example>>

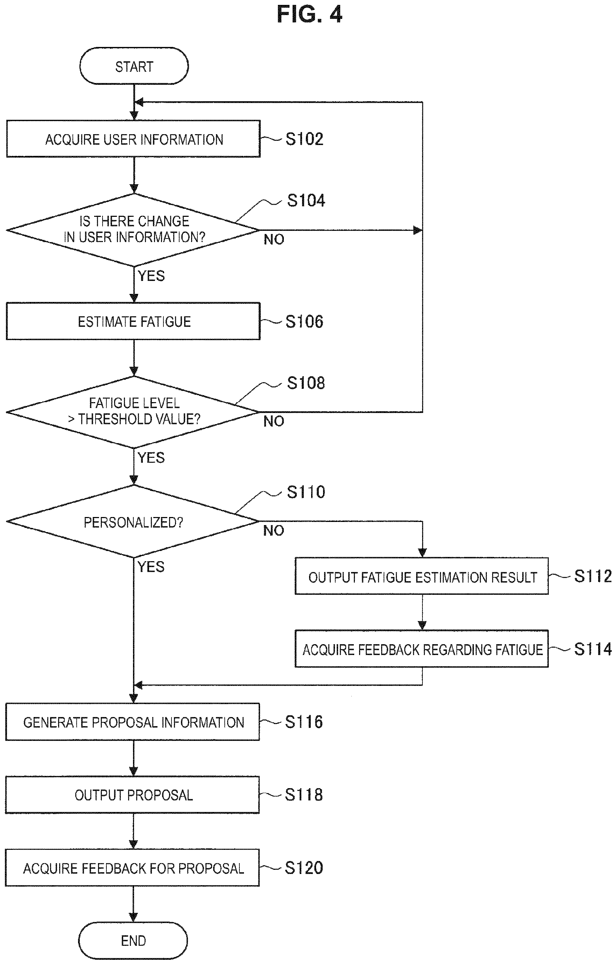

Subsequently, an operation example of the information processing apparatus 1 according to the present embodiment will be described. Hereinafter, first of all, a flow of processing will be described with reference to FIG. 4, and then, a specific example of response control according to the present embodiment will be described.

<2-1. Flow of processing>

FIG. 4 is a flowchart diagram illustrating an example of a flow of processing of the information processing apparatus 1 according to the present embodiment. As illustrated in FIG. 4, first of all, the information input unit acquires user information (SI02). In a case where there is no change in the user information (NO in S104), the processing returns to step S102. On the other hand, in a case where there is a change in the user information (YES in S104), the fatigue estimation unit 13 estimates fatigue of each fatigue type on the basis of the user information (S106). In a case where an estimated fatigue level is equal to or smaller than a threshold value (NO in S108), the processing returns to step S102.

In a case where the estimated fatigue level is larger than the threshold value (YES in S108), it is determined whether the fatigue estimator has been sufficiently personalized or not (S110). In a case where it is determined that the fatigue estimator has not been sufficiently personalized (NO in S110), the output control unit 15 causes the fatigue level of each fatigue type (fatigue estimation result) obtained in step S106, to be output (S112). Subsequently, a user feedback regarding fatigue is acquired (S114). On the other hand, in a case where it is determined that the fatigue estimator has been sufficiently personalized (YES in S110), the processes in steps S112 and S114 are skipped, and the processing proceeds to step S116.

In step S116, the proposal generation unit 14 generates proposal information on the basis of the fatigue level of each type. Subsequently, the output control unit 15 causes proposal to be output on the basis of the proposal information (S118). Subsequently, a user feedback for the proposal is acquired (S120).

<2-2. Specific example of improvement proposal>

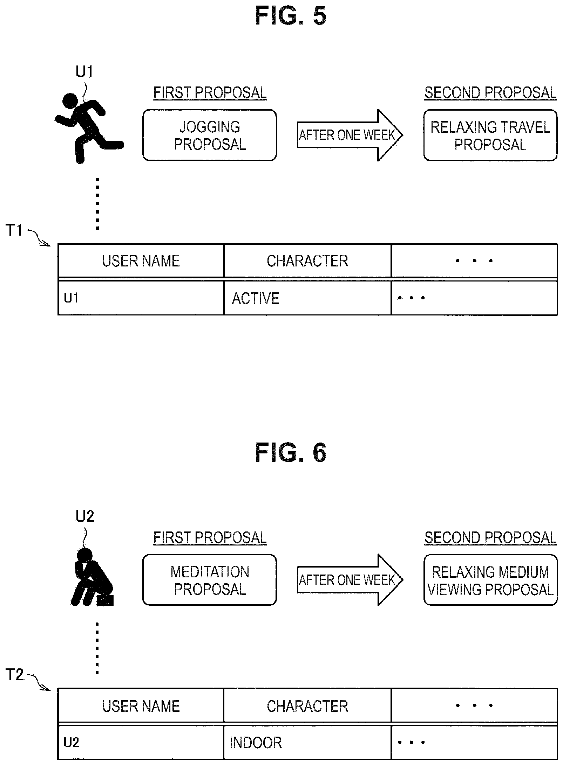

An example of a flow of processing of the information processing apparatus 1 according to the present embodiment has been described above. Subsequently, a specific example regarding the above-described improvement proposal will be described with reference to FIGS. 5 and 6.

FIG. 5 is a conceptual diagram illustrating a specific example of improvement proposal for a user U1. In addition, FIG. 6 is a conceptual diagram illustrating a specific example of improvement proposal for a user U2. FIGS. 5 and 6 illustrate examples in which, for both of the user U1 and the user U2, a fatigue level is estimated to be "medium" in FIG. 3 and a fatigue type is estimated to be mental fatigue, and a week after the first improvement proposal has been performed, a fatigue level is estimated to be "medium" again and a fatigue type is estimated to be mental fatigue again.

Tables T1 and T2 respectively illustrated in FIGS. 5 and 6 are examples of user profiles related to the respective users U1 and U2. As illustrated in FIG. 5, for the user U1 having an "active" character, jogging proposal is output as the first proposal. On the other hand, as illustrated in FIG. 6, for the user U2 having an "indoor" character, meditation proposal is output as the first proposal. As in these examples, the proposal generation unit 14 can generate proposal information pieces different for the respective users in accordance with the user profiles even if the estimated fatigue type and the fatigue level are the same. With this configuration, it becomes easier to perform proposal more preferable for the user.

In addition, as illustrated in FIG. 5, for the user U1, relaxing travel proposal is output as the second proposal. On the other hand, as illustrated in FIG. 6, for the user U2, relaxing medium viewing proposal is output as the second proposal. As in these examples, the proposal generation unit 14 can generate proposal information different from previously-generated proposal information in accordance with the history of proposal information even if the estimated fatigue type and the fatigue level are the same. With this configuration, mannerism of proposal can be suppressed.

<<3. Modified example>>

An embodiment of the present disclosure has been described above. Hereinafter, several modified examples of the embodiment of the present disclosure will be described. Note that the modified examples to be described below may be independently applied to the embodiment of the present disclosure, or may be applied to the embodiment of the present disclosure in combination. In addition, each modified example may be applied in place of the configuration described in the embodiment of the present disclosure, or may be applied in addition to the configuration described in the embodiment of the present disclosure.

<3-1. First modified example>

In the above-described embodiment, the description has been given of an example in which each function described with reference to FIG. 1 is included in a single apparatus (the information processing apparatus 1), but the present technology is not limited to this example. Hereinafter, as a first modified example, the description will be given of an example in which the above-described effect is realized by cooperation between a plurality of apparatuses.

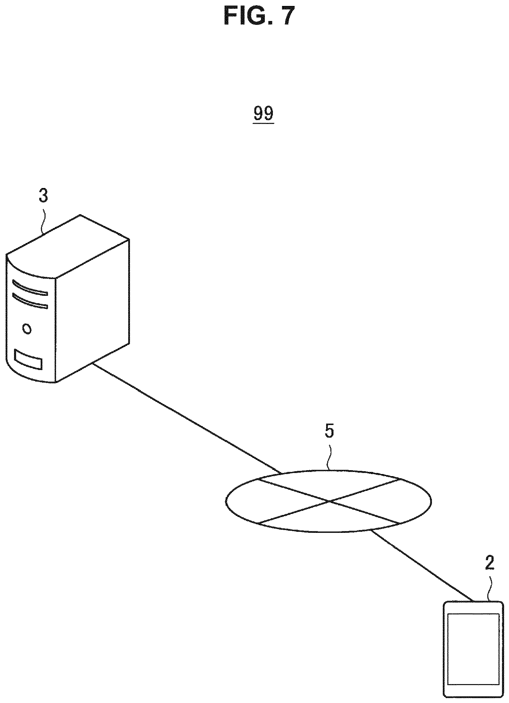

FIG. 7 is an explanatory diagram illustrating a configuration of an information processing system 99 according to the modified example. As illustrated in FIG. 7, the information processing system 99 according to the modified example includes a client terminal 2, a server 3, and a communication network 5.

FIG. 7 illustrates an example in which the client terminal 2 is a smartphone, but the client terminal 2 is not limited to a smartphone, and may be a PC, a robot, a wearable device, or the like.

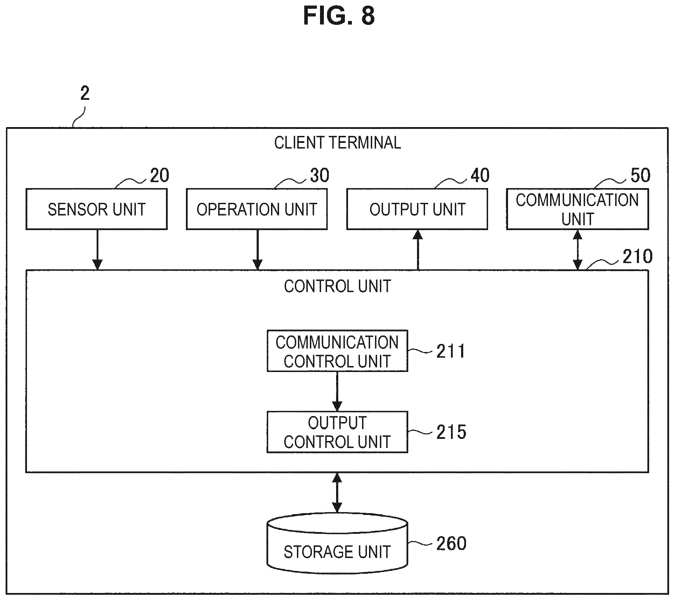

FIG. 8 is a block diagram illustrating a configuration example of the client terminal 2. As illustrated in FIG. 8, the client terminal 2 according to the application example is an information processing apparatus including a control unit 210, the sensor unit 20, the operation unit 30, the output unit 40, the communication unit 50, and a storage unit 260. Note that, because configurations substantially similar to the configurations illustrated in FIG. 1, among configurations illustrated in FIG. 8, are denoted with the same reference numerals, the description will be given while appropriately omitting such configurations.

The control unit 210 illustrated in FIG. 8 controls each configuration of the client terminal 2. As illustrated in FIG. 8, the control unit 210 according to the modified example has functions as a communication control unit 211 and an output control unit 215.

The communication control unit 211 controls communication performed by the communication unit 50. For example, by the communication control unit 211 controlling the communication unit 50, it becomes possible for the communication unit 50 to transmit user information to the server 3, and receive proposal information for fatigue improvement from the server 3.

The output control unit 215 (an example of a processing unit) performs control processing of an output performed by the output unit 40. For example, the output control unit 215 performs output control processing of proposal that is based on proposal information that the communication control unit 211 causes the communication unit 50 to receive.

Similarly to the storage unit 60, the storage unit 260 stores programs and parameters for each configuration of the client terminal 2 functioning. In addition, the storage unit 260 may store information regarding an application executed by the client terminal 2, user information obtained in the past, a user profile, and the like, but needs not store the history of proposal information.

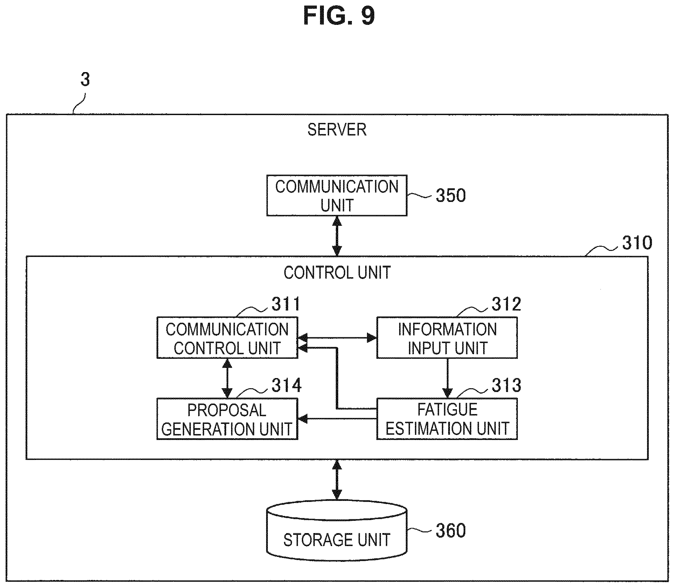

FIG. 9 is a block diagram illustrating a configuration example of the server 3. As illustrated in FIG. 9, the server 3 according to the application example is an information processing apparatus including a control unit 310, a communication unit 350, and a storage unit 360.

The control unit 310 controls each configuration of the server 3. As illustrated in FIG. 9, the control unit 310 according to the application example has functions as a communication control unit 311, an information input unit 312, a fatigue estimation unit 313, and a proposal generation unit 314.

The communication control unit 311 controls communication performed by the communication unit 50. For example, by the communication control unit 311 controlling the communication unit 350, it becomes possible for the communication unit 350 to receive user information from the client terminal 2, and transmit proposal information for fatigue improvement to the client terminal 2.

Similarly to the information input unit 12 described with reference to FIG. 1, the information input unit 312 acquires user information from the communication control unit 311 and the storage unit 360, and inputs the acquired user information to the fatigue estimation unit 313 in a case where there is a change in the user information. Because the server 3 according to the modified example does not include a sensor unit as illustrated in FIG. 9, the information input unit 312 acquires user information obtained by sensing, from another apparatus (e.g. the client terminal 2) including a sensor unit.

Similarly to the fatigue estimation unit 13 described with reference to FIG. 1, in a case where user information is input from the information input unit 312, the fatigue estimation unit 313 (an example of an acquisition unit) estimates and acquires a fatigue level of each fatigue type on the basis of the user information. Because the server 3 according to the modified example does not include an output unit and an output control unit as illustrated in FIG. 9, the fatigue estimation unit 313 may provide a fatigue estimation result to the client terminal 2 via the communication control unit 311 instead of providing a fatigue estimation result to an output control unit. In addition, information related to a feedback regarding fatigue is transmitted from the client terminal 2 to the server 3.

Similarly to the proposal generation unit 14 described with reference to FIG. 1, the proposal generation unit 314 generates proposal information related to proposal for improving fatigue, on the basis of a fatigue level of each fatigue type that has been provided by the fatigue estimation unit 13. Note that, because the server 3 according to the modified example does not include an output unit and an output control unit as illustrated in FIG. 9, the proposal generation unit 314 may provide the proposal information to the client terminal 2 via the communication control unit 311 instead of providing the proposal information to an output control unit. In addition, information related to a feedback for the proposal is transmitted from the client terminal 2 to the server 3.

The communication unit 350 is controlled by the communication control unit 311, and performs information communication with another apparatus.

Similarly to the storage unit 60, the storage unit 360 stores programs and parameters for each configuration of the server 3 functioning. In addition, the storage unit 360 may store information regarding an application executed by the server 3, user information obtained in the past, a user profile, the history of proposal information, and the like.

The communication network 5 is a wired or wireless transmission path of information transmitted from an apparatus or a system that is connected to the communication network 5. For example, the communication network 5 may include a public circuit network such as the Internet, a telephone circuit network, or a satellite communication network, various local area networks (LANs) including Ethernet (registered trademark), a wide area network (WAN), and the like. In addition, the communication network 5 may include a dedicated circuit network such as an internet protocol-virtual private network (IP-VPN).

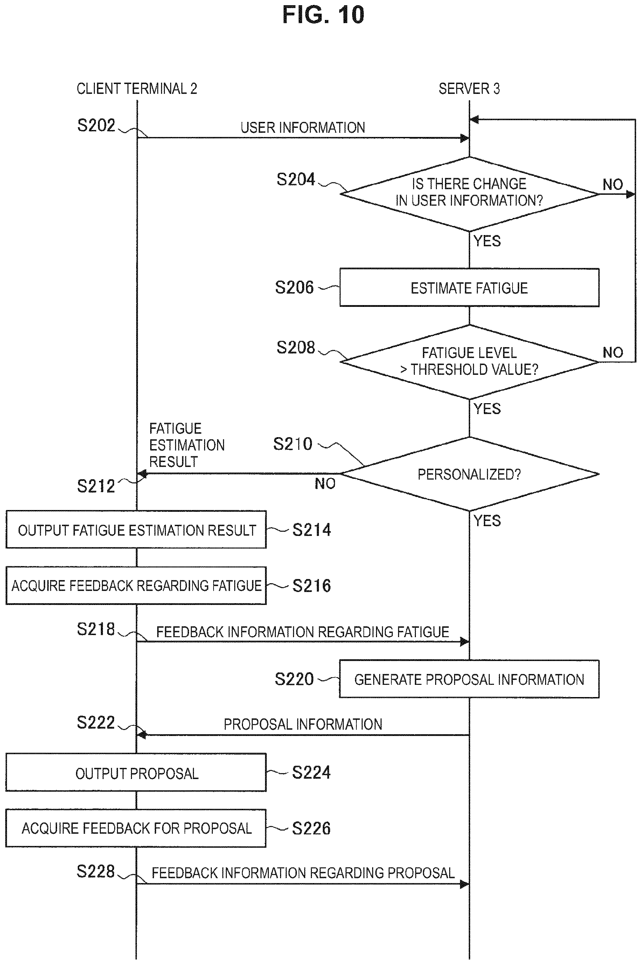

The configuration example of the information processing system 99 according to the modified example has been described above. Subsequently, an operation example of the information processing system 99 according to the modified example will be described with reference to FIG. 10. FIG. 10 is a sequence diagram illustrating an operation example of the information processing system 99 according to the modified example.

First of all, user information is transmitted from the client terminal 2 to the server 3 (S202). Subsequently, in the server 3, it is determined whether there is a change in the user information or not (S204). In a case where there is no change in the user information (NO in S204), the processing returns to step S202. On the other hand, in a case where there is a change in the user information (YES in S204), the server 3 estimates fatigue of each fatigue type on the basis of the user information (S206). In a case where an estimated fatigue level is equal to or smaller than a threshold value (NO in S208), the processing returns to step S202.

In a case where the estimated fatigue level is larger than the threshold value (YES in S208), it is determined whether the fatigue estimator has been sufficiently personalized or not (S210). In a case where it is determined that the fatigue estimator has not been sufficiently personalized (NO in S210), the server 3 transmits the fatigue level of each fatigue type (fatigue estimation result) obtained in step S206, to the client terminal 2 (S212). Subsequently, in the client terminal 2, the fatigue estimation result is output. Subsequently, in the client terminal 2, a user feedback regarding fatigue is acquired (S216), and information related to the user feedback regarding fatigue is transmitted from the client terminal 2 to the server 3 (S218).

On the other hand, in a case where it is determined that the fatigue estimator has been sufficiently personalized (YES in S210), the processed in steps S212 to S218 are skipped, and the processing proceeds to step S220.

In step S220, the server 3 generates proposal information on the basis of a fatigue level of each type. Subsequently, the generated proposal information is transmitted from the server 3 to the client terminal 2 (S222). The client terminal 2 that has received the proposal information causes proposal to be output on the basis of the proposal information (S224). Subsequently, in the client terminal 2, a user feedback for the proposal is acquired (S226). The client terminal 2 transmits information related to the acquired user feedback for the proposal, to the server 3 (S228).

As the first modified example, the above description has been given of an example in which functions similar to the functions according to the embodiment of the present disclosure are realized by cooperation between a plurality of apparatuses. Note that a combination of functions included in each apparatus is not limited to the above combination. For example, a client terminal may further have functions of an information input unit and a fatigue estimation unit, and transmit a fatigue level of each fatigue type to a server. In this case, a communication control unit included in the server can function as an acquisition unit that acquires a fatigue level.

<3-2. Second modified example>

In the above-described embodiment, the description has been given of an example in which a user feedback regarding fatigue is obtained through a screen as illustrated in FIG. 2, and a fatigue estimation result is corrected, but the present technology is not limited to this example. For example, by the user inputting answers to predetermined questions, a fatigue level of each fatigue type may be corrected more accurately. As an example, examples of questions regarding physical fatigue are listed in Table 1 and examples of questions regarding mental fatigue are listed in Table 2.

[Table 1]

Table 1: Examples of questions regarding physical fatigue

| Have a slight fever |
| --- |
| Get very tired by light exercise or work |
| Have muscular pain |
| Have an enlarged lymph node |
| Feel tired or woozy |
| Remain tired even after one night sleep |
| Have throat pain |
| Have joint pain |
| Have no power in body recently |
| Have a headache, feel heavy-headed |

[Table 2]

Table 2: Examples of questions regarding mental fatigue

| Sleep poorly |
| --- |

(continued)

| |
|---|
| Cannot remember small matters |
| Feel depressed |
| Often dazed |
| Inevitably sleep too much |
| Worried about physical condition |
| Thinking power is declining |
| Concentration power is declining |
| Unwilling to work |
| Sometimes feel too bright and dizzy |

For each item in Table 1 and Table 2, the user may input an answer using YES or NO or may input an answer using a numerical value. In addition, by correcting a fatigue level on the basis of information regarding an answer to a question, it becomes possible for a fatigue estimation unit to acquire a more accurate corrected fatigue level.

<<4. Hardware configuration example>>

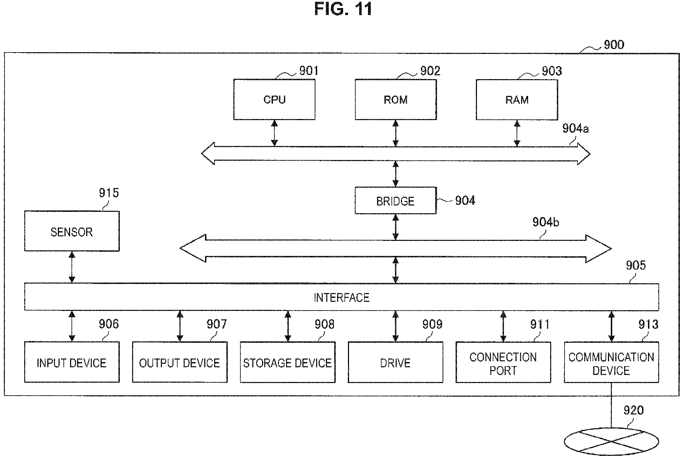

The embodiment of the present disclosure has been described hitherto. Finally, a hardware configuration of an information processing apparatus according to the present embodiment of the present disclosure will be described with reference to FIG. 11. FIG. 11 is a block diagram illustrating an example of the hardware configuration of the information processing apparatus according to the present embodiment of the present disclosure. Meanwhile, an information processing apparatus 900 illustrated in FIG. 11 may realize the information processing apparatus 1 illustrated in FIG. 1, the client terminal 2 illustrated in FIG. 8, and the server 3 illustrated in FIG. 9, for example. Information processing by the information processing apparatus 1, the client terminal 2, and the server 3 according to the present embodiment is realized according to cooperation between software and hardware described below.

As illustrated in FIG. 11, the information processing apparatus 900 includes a central processing unit (CPU) 901, a read only memory (ROM) 902, a random access memory (RAM) 903, and a host bus 904a. In addition, the information processing apparatus 900 includes a bridge 904, an external bus 904b, an interface 905, an input device 906, an output device 907, a storage device 908, a drive 909, a connection port 911, a communication device 913, and a sensor 915. The information processing apparatus 900 may include a processing circuit such as a DSP or an ASIC instead of the CPU 901 or along therewith.

The CPU 901 functions as an arithmetic processing device and a control device and controls the overall operation in the information processing apparatus 900 according to various programs. Further, the CPU 901 may be a microprocessor. The ROM 902 stores programs, operation parameters, and the like used by the CPU 901. The RAM 903 temporarily stores programs used in execution of the CPU 901, parameters appropriately changed in the execution, and the like. The CPU 901 may form the control unit 10, the control unit 210, the control unit 310, and the like, for example.

The CPU 901, the ROM 902, and the RAM 903 are connected by the host bus 904a including a CPU bus and the like. The host bus 904a is connected with the external bus 904b such as a peripheral component interconnect/interface (PCI) bus via the bridge 904. Further, the host bus 904a, the bridge 904, and the external bus 904b are not necessarily separately configured and such functions may be mounted in a single bus.

The input device 906 is realized by a device through which a user inputs information, such as a mouse, a keyboard, a touch panel, a button, a microphone, a switch, and a lever, for example. In addition, the input device 906 may be a remote control device using infrared ray or other electric waves, or external connection equipment such as a cellular phone or a PDA corresponding to an operation of the information processing apparatus 900, for example. Furthermore, the input device 906 may include an input control circuit or the like which generates an input signal on the basis of information input by the user using the aforementioned input means and outputs the input signal to the CPU 901, for example. The user of the information processing apparatus 900 may input various types of data or order a processing operation for the information processing apparatus 900 by operating the input device 906. The input device 906 may form the operation unit 30.

The output device 907 is formed by a device that may visually or aurally notify the user of acquired information. As such devices, there are a display device such as a CRT display device, a liquid crystal display device, a plasma display device, an EL display device, or a lamp, a sound output device such as a speaker and a headphone, a printer device, and the like. The output device 907 outputs results acquired through various processes performed by the information processing apparatus 900, for example. Specifically, the display device visually displays results acquired through various processes performed by the information processing apparatus 900 in various forms such as text, images, tables, and graphs. On the other hand, the sound output device converts audio signals including reproduced sound data, audio data, and the like into analog signals and aurally outputs the analog signals. The output device 907 may form the output unit 40, for example.

The storage device 908 is a device for data storage, formed as an example of a storage unit of the information processing apparatus 900. For example, the storage device 908 is realized by a magnetic storage device such as an HDD, a semiconductor storage device, an optical storage device, a magneto-optical storage device, or the like. The storage device 908 may include a storage medium, a recording device for recording data on the storage medium, a reading device for reading data from the storage medium, a deletion device for deleting data recorded on the storage medium, and the like. The storage device 908 stores programs and various types of data executed by the CPU 901, various types of data acquired from the outside, and the like. The storage device 908 may form the storage unit 60, the storage unit 260, and the storage unit 360, for example.

The drive 909 is a reader/writer for storage media and is included in or externally attached to the information processing apparatus 900. The drive 909 reads information recorded on a removable storage medium such as a magnetic disc, an optical disc, a magneto-optical disc, or a semiconductor memory mounted thereon, and outputs the information to the RAM 903. In addition, the drive 909 may write information regarding the removable storage medium.

The connection port 911 is an interface connected with external equipment and is a connector to the external equipment through which data may be transmitted through a universal serial bus (USB) and the like, for example.

The communication device 913 is a communication interface formed by a communication device for connection to a network 920 or the like, for example. The communication device 913 is a communication card or the like for a wired or wireless local area network (LAN), long term evolution (LTE), Bluetooth (registered trademark), or wireless USB (WUSB), for example. In addition, the communication device 913 may be a router for optical communication, a router for asymmetric digital subscriber line (ADSL), various communication modems, or the like. For example, the communication device 913 may transmit/receive signals and the like to/from the Internet and other communication apparatuses according to a predetermined protocol such as, for example, TCP/IP.

Further, the network 920 is a wired or wireless transmission path of information transmitted from devices connected to the network 920. For example, the network 920 may include a public circuit network such as the Internet, a telephone circuit network, or a satellite communication network, various local area networks (LANs) including Ethernet (registered trademark), a wide area network (WAN), and the like. In addition, the network 920 may include a dedicated circuit network such as an internet protocol-virtual private network (IP-VPN).

The sensor 915 corresponds to various types of sensors such as an acceleration sensor, a gyro sensor, a geomagnetic sensor, a light sensor, a sound sensor, a distance measuring sensor, and a force sensor, for example. The sensor 915 acquires information regarding a state of the information processing apparatus 900 itself, such as an attitude and a movement speed of the information processing apparatus 900, and information regarding a surrounding environment of the information processing apparatus 900, such as brightness and noise of the periphery of the information processing apparatus 900. In addition, the sensor 915 may include a GPS sensor that receives a GPS signal, and measures latitude, longitude, and altitude of the device. The sensor 915 may form, for example, the sensor unit 20.

Hereinbefore, an example of a hardware configuration capable of realizing the functions of the information processing apparatus 900 according to this embodiment is shown. The respective components may be implemented using universal members, or may be implemented by hardware specific to the functions of the respective components. Accordingly, according to a technical level at the time when the embodiments are executed, it is possible to appropriately change hardware configurations to be used.

In addition, a computer program for realizing each of the functions of the information processing apparatus 900 according to the present embodiment as described above may be created, and may be mounted in a PC or the like. Furthermore, a computer-readable recording medium on which such a computer program is stored may be provided. The recording medium is a magnetic disc, an optical disc, a magneto-optical disc, a flash memory, or the like, for example. Further, the computer program may be delivered through a network, for example, without using the recording medium. In addition, the above-described computer program may be distributed through, for example, a network without using a recording medium. In addition, the number of computers executing the computer program is not particularly limited. For example, a plurality of computers (for example, a plurality of servers or the like) may execute the computer program in association with each other.

<<5. Conclusion>>

As described above, according to an embodiment of the present disclosure, proposal for improving fatigue can be performed.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

In addition, steps in the above-described embodiment need not be always processed in chronological order in accordance with the order described as a flowchart diagram or a sequence diagram. For example, steps in the processes in the above-described embodiment may be processed in an order different from the order described as a flowchart diagram or a sequence diagram, or may be concurrently processed.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.

(1) An information processing apparatus including:

an acquisition unit configured to acquire a fatigue level of a user; and
a proposal generation unit configured to generate proposal information for fatigue improvement on the basis of the fatigue level.

(2) The information processing apparatus according to (1), in which the proposal generation unit generates the proposal information further on the basis of a fatigue type.
(3) The information processing apparatus according to (1) or (2), in which the proposal generation unit generates the proposal information further on the basis of a user profile related to the user.
(4) The information processing apparatus according to any one of (1) to (3), in which the proposal generation unit generates the proposal information further on the basis of a history of generated proposal information.
(5) The information processing apparatus according to (4), in which the proposal generation unit generates proposal information so as not to include proposal information included in the history.
(6) The information processing apparatus according to (4) or (5), in which the history includes information as to whether proposal that is based on the proposal information has been actually implemented by the user or not.
(7) The information processing apparatus according to any one of (1) to (6), in which the fatigue level is acquired on the basis of user information regarding the user.
(8) The information processing apparatus according to (7), in which the fatigue level is estimated by inputting the user information to a fatigue estimator.
(9) The information processing apparatus according to (8), in which the estimated fatigue level is corrected on the basis of feedback from the user regarding fatigue.
(10) The information processing apparatus according to (9), in which the fatigue estimator is personalized on the basis of a corrected fatigue level.
(11) The information processing apparatus according to (10), in which the fatigue estimator is personalized by learning the corrected fatigue level.
(12) The information processing apparatus according to (10) or (11), in which the feedback is acquired in a case where it is determined that the fatigue estimator has not been sufficiently personalized, and the feedback is not acquired in a case where it is determined that the fatigue estimator has been sufficiently personalized.
(13) The information processing apparatus according to any one of (7) to (12), in which the user information includes at least any one of position information of the user, information regarding an application used by the user, an action history of the user, or state information of the user.
(14) An information processing apparatus including:

a communication unit configured to transmit user information regarding a user, and receive proposal information for fatigue improvement; and
a processing unit configured to perform processing that is based on the proposal information.

(15) An information processing method including:

acquiring a fatigue level; and
generating, by a processor, proposal information for fatigue improvement on the basis of the fatigue level.

(16) An information processing method including:

transmitting user information regarding a user, and receiving proposal information for fatigue improvement; and

performing, by a processor, processing that is based on the proposal information.

Reference Signs List

| | |
|---|---|
| 1 | information processing apparatus |
| 2 | client terminal |
| 3 | server |
| 5 | communication network |
| 10 | control unit |
| 11 | communication control unit |
| 12 | information input unit |
| 13 | fatigue estimation unit |
| 14 | proposal generation unit |
| 15 | output control unit |
| 20 | sensor unit |
| 30 | operation unit |
| 40 | output unit |
| 50 | communication unit |
| 60 | storage unit |
| 99 | information processing system |

**Claims**

1. An information processing apparatus comprising:

   an acquisition unit configured to acquire a fatigue level of a user; and
   a proposal generation unit configured to generate proposal information for fatigue improvement on a basis of the fatigue level.

2. The information processing apparatus according to claim 1, wherein the proposal generation unit generates the proposal information further on a basis of a fatigue type.

3. The information processing apparatus according to claim 1, wherein the proposal generation unit generates the proposal information further on a basis of a user profile related to the user.

4. The information processing apparatus according to claim 1, wherein the proposal generation unit generates the proposal information further on a basis of a history of generated proposal information.

5. The information processing apparatus according to claim 4, wherein the proposal generation unit generates proposal information so as not to include proposal information included in the history.

6. The information processing apparatus according to claim 4, wherein the history includes information as to whether proposal that is based on the proposal information has been actually implemented by the user or not.

7. The information processing apparatus according to claim 1, wherein the fatigue level is acquired on a basis of user information regarding the user.

8. The information processing apparatus according to claim 7, wherein the fatigue level is estimated by inputting the user information to a fatigue estimator.

9. The information processing apparatus according to claim 8, wherein the estimated fatigue level is corrected on a basis of feedback from the user regarding fatigue.

10. The information processing apparatus according to claim 9, wherein the fatigue estimator is personalized on a basis of a corrected fatigue level.

**11.** The information processing apparatus according to claim 10, wherein the fatigue estimator is personalized by learning the corrected fatigue level.

**12.** The information processing apparatus according to claim 10, wherein the feedback is acquired in a case where it is determined that the fatigue estimator has not been sufficiently personalized, and the feedback is not acquired in a case where it is determined that the fatigue estimator has been sufficiently personalized.

**13.** The information processing apparatus according to claim 7, wherein the user information includes at least any one of position information of the user, information regarding an application used by the user, an action history of the user, or state information of the user.

**14.** An information processing apparatus comprising:

a communication unit configured to transmit user information regarding a user, and receive proposal information for fatigue improvement; and
a processing unit configured to perform processing that is based on the proposal information.

**15.** An information processing method comprising:

acquiring a fatigue level; and
generating, by a processor, proposal information for fatigue improvement on a basis of the fatigue level.

**16.** An information processing method comprising:

transmitting user information regarding a user, and receiving proposal information for fatigue improvement; and
performing, by a processor, processing that is based on the proposal information.

# FIG. 1

1

INFORMATION PROCESSING APPARATUS

| 20 | 30 | 40 | 50 |
|---|---|---|---|
| SENSOR UNIT | OPERATION UNIT | OUTPUT UNIT | COMMUNICATION UNIT |

10

CONTROL UNIT

| 11 | 12 |
|---|---|
| COMMUNICATION CONTROL UNIT | INFORMATION INPUT UNIT |

| 14 | 13 |
|---|---|
| PROPOSAL GENERATION UNIT | FATIGUE ESTIMATION UNIT |

15

OUTPUT CONTROL UNIT

STORAGE UNIT 60

# FIG. 2

G1

YOU LOOK TIRED RECENTLY.
IF YOU SELECT ANY FATIGUE OF THE FOLLOWING,
IMPROVEMENT PROPOSAL WILL BE PROVIDED.

G11 ——— 1. MENTAL FATIGUE

G12 ——— 2. BRAIN FATIGUE

G13 ——— 3. PHYSICAL FATIGUE

G14 ——— 4. NOT TIRED

# FIG. 3

# FIG. 4

```
                    ┌─────────────┐
                    │    START    │
                    └──────┬──────┘
                           │
                           ▼
            ┌──────────────────────────────┐
            │   ACQUIRE USER INFORMATION    │ ～ S102
            └──────────────┬────────────────┘
                           │
                           ▼
                      ╱─────────╲
                    ╱  IS THERE   ╲  ～ S104
                   ╱    CHANGE      ╲
                  ╱ IN USER INFORMATION?╲ ───── NO ─────┐
                   ╲               ╱    NO              │
                    ╲             ╱                     │
                      ╲─────────╱                       │
                         │ YES                          │
                         ▼                              │
            ┌──────────────────────────────┐           │
            │       ESTIMATE FATIGUE        │ ～ S106   │
            └──────────────┬────────────────┘           │
                           │                            │
                           ▼                            │
                      ╱─────────╲                       │
                    ╱FATIGUE LEVEL╲ ～ S108              │
                   ╱ > THRESHOLD   ╲ ──── NO ───────────┘
                   ╲    VALUE?     ╱  NO
                    ╲             ╱
                      ╲─────────╱
                         │ YES
                         ▼
                      ╱─────────╲
                    ╱            ╲ ～ S110
                   ╱ PERSONALIZED? ╲ ──── NO ────┐
                   ╲              ╱  NO           │
                    ╲            ╱                ▼
                      ╲────────╱      ┌──────────────────────────────┐
                         │ YES        │OUTPUT FATIGUE ESTIMATION RESULT│ ～ S112
                         │            └──────────────┬───────────────┘
                         │                           │
                         │                           ▼
                         │            ┌──────────────────────────────┐
                         │            │ACQUIRE FEEDBACK REGARDING FATIGUE│ ～ S114
                         │            └──────────────┬───────────────┘
                         │◄──────────────────────────┘
                         ▼
            ┌──────────────────────────────┐
            │  GENERATE PROPOSAL INFORMATION │ ～ S116
            └──────────────┬────────────────┘
                           │
                           ▼
            ┌──────────────────────────────┐
            │        OUTPUT PROPOSAL         │ ～ S118
            └──────────────┬────────────────┘
                           │
                           ▼
            ┌──────────────────────────────┐
            │  ACQUIRE FEEDBACK FOR PROPOSAL │ ～ S120
            └──────────────┬────────────────┘
                           │
                           ▼
                    ┌─────────────┐
                    │     END     │
                    └─────────────┘
```

# FIG. 5

U1

FIRST PROPOSAL

JOGGING
PROPOSAL

AFTER ONE WEEK

SECOND PROPOSAL

RELAXING TRAVEL
PROPOSAL

T1

| USER NAME | CHARACTER | · · · |
|-----------|-----------|-------|
| U1 | ACTIVE | · · · |

# FIG. 6

U2

FIRST PROPOSAL

MEDITATION
PROPOSAL

AFTER ONE WEEK

SECOND PROPOSAL

RELAXING MEDIUM
VIEWING PROPOSAL

T2

| USER NAME | CHARACTER | · · · |
|-----------|-----------|-------|
| U2 | INDOOR | · · · |

# FIG. 7

<u>99</u>

# FIG. 8

2

```
CLIENT TERMINAL

        20              30              40              50
  SENSOR UNIT    OPERATION UNIT   OUTPUT UNIT    COMMUNICATION
                                                      UNIT

                                                          210
                        CONTROL UNIT

                    COMMUNICATION
                    CONTROL UNIT        211

                        OUTPUT
                    CONTROL UNIT        215

                     STORAGE UNIT       260
```

# FIG. 9

3

SERVER

| COMMUNICATION UNIT | ～350 |

～310

CONTROL UNIT

～311
COMMUNICATION CONTROL UNIT

～312
INFORMATION INPUT UNIT

～314
PROPOSAL GENERATION UNIT

～313
FATIGUE ESTIMATION UNIT

STORAGE UNIT ～360

# FIG. 10

CLIENT TERMINAL 2                                                    SERVER 3

S202 ———— USER INFORMATION ————→

S204 ⟨ IS THERE CHANGE IN USER INFORMATION? ⟩ —— NO →

YES

S206 — [ ESTIMATE FATIGUE ]

S208 ⟨ FATIGUE LEVEL > THRESHOLD VALUE? ⟩ —— NO →

YES

FATIGUE ESTIMATION RESULT

S210 ⟨ PERSONALIZED? ⟩

S212 ←———— NO ————

S214 — [ OUTPUT FATIGUE ESTIMATION RESULT ]

YES

S216 — [ ACQUIRE FEEDBACK REGARDING FATIGUE ]

S218 ———— FEEDBACK INFORMATION REGARDING FATIGUE ————→

S220 — [ GENERATE PROPOSAL INFORMATION ]

S222 ←———— PROPOSAL INFORMATION ————

S224 — [ OUTPUT PROPOSAL ]

S226 — [ ACQUIRE FEEDBACK FOR PROPOSAL ]

S228 ———— FEEDBACK INFORMATION REGARDING PROPOSAL ————→

## FIG. 11

## INTERNATIONAL SEARCH REPORT

| International application No. |
| --- |
| PCT/JP2017/026329 |

**A. CLASSIFICATION OF SUBJECT MATTER**
*G06Q50/22*(2012.01)i, *A61B5/16*(2006.01)i

According to International Patent Classification (IPC) or to both national classification and IPC

**B. FIELDS SEARCHED**

Minimum documentation searched (classification system followed by classification symbols)
G06Q50/22, A61B5/16

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2014/156076 A1 (Panasonic Intellectual Property Corporation of America), 02 October 2014 (02.10.2014), paragraphs [0010], [0037], [0043] to [0045], [0051], [0056] to [0203], [0235] to [0269]; fig. 1 to 27, 32 to 34<br>& US 2015/0169834 A1<br>paragraphs [0066], [0103], [0109] to [0111], [0117], [0122] to [0316], [0352] to [0388]; fig. 1 to 27, 32 to 34 | 1-2,7-13,15<br>3-6,14,16 |

☒ Further documents are listed in the continuation of Box C. ☐ See patent family annex.

| | |
| --- | --- |
| *    Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| 01 September 2017 (01.09.17) | 12 September 2017 (12.09.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**INTERNATIONAL SEARCH REPORT**

International application No.

PCT/JP2017/026329

C (Continuation).　DOCUMENTS CONSIDERED TO BE RELEVANT

| Category* | Citation of document, with indication, where appropriate, of the relevant passages | Relevant to claim No. |
|---|---|---|
| Y | WO 2015/107710 A1  (Nintendo Co., Ltd.),<br>23 July 2015 (23.07.2015),<br>paragraphs [0080], [0082], [0151] to [0156],<br>[0170] to [0173], [0193], [0201]; fig. 1, 9, 12,<br>14, 15<br>& US 2016/0328533 A1<br>paragraphs [0174], [0176], [0273] to [0278],<br>[0292] to [0295], [0317], [0325]; fig. 1, 9, 12,<br>14, 15<br>& EP 3096235 A1 | 3-6,14,16 |
| A | JP 2014-527219 A  (Saudi Arabian Oil Co.),<br>09 October 2014 (09.10.2014),<br>entire text; all drawings<br>& US 2013/0012802 A1　& EP 2729065 A2<br>& AU 2012279113 A1　& CA 2840775 A1<br>& CN 103764022 A | 1-16 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP H10024017 A